(19) Europäisches Patentamt
European Patent Office
Office européen des brevets

(11) **EP 1 832 233 A1**

(12) **EUROPEAN PATENT APPLICATION**
published in accordance with Art. 158(3) EPC

(43) Date of publication:
**12.09.2007 Bulletin 2007/37**

(21) Application number: **05819627.0**

(22) Date of filing: **26.12.2005**

(51) Int Cl.:
**A61B 8/00** (2006.01)

(86) International application number:
**PCT/JP2005/023797**

(87) International publication number:
**WO 2006/068271 (29.06.2006 Gazette 2006/26)**

(84) Designated Contracting States:
**DE FR GB NL**

(30) Priority: **24.12.2004 JP 2004374860**

(71) Applicants:
• **Yd, Ltd.**
**Ikoma-city**
**Nara 6300243 (JP)**
• **Yoshida, Kiyoshi**
**Kurashiki-shi**
**Okayama 7010192 (JP)**
• **Seikotec Co., Ltd.**
**Higashi-ku, Fukuoka-shi**
**Fukuoka 8130062 (JP)**
• **Watanabe, Nozomi**
**Kurashiki-shi**
**Okayama 7010192 (JP)**

• **Ogasawara, Yasuo**
**Kurashiki-shi**
**Okayama 7010192 (JP)**

(72) Inventors:
• **SAKURAI, Masashi**
**uoka 8100063; (JP)**
• **WATANABE, N.;c/o Dep. of Cardiology;**
**Kawasaki**
**Okayama 7010192 (JP)**
• **OGASAWARA, Y.**
**c/o Dep. of Med. Engineering**
**Kurashiki-shi**
**Okayama 7010192 (JP)**

(74) Representative: **Banzer, Hans-Jörg**
**Kraus & Weisert,**
**Thomas-Wimmer-Ring 15**
**80539 München (DE)**

(54) **CARDIAC VALVE DATA MEASURING METHOD AND DEVICE**

(57)    An object of the present invention is to acquire information regarding the cardiac valve required in the clinical field, such as the tenting volume, tenting area, tenting height of the mitral valve of the heart, the area, the circumferential length, and height (the difference between the highest portion and the lowest portion) of the mitral annulus, etc.

A method of obtaining a three-dimensional cardiac-valve image for measuring clinically required data regarding the cardiac valve, in which method a three-dimensional echocardiogram is created from two-dimensional echocardiograms obtained through scanning by means of an echocardiograph, and the three-dimensional cardiac-valve image is automatically extracted from the three-dimensional echocardiogram by computer processing. The method is **characterized in that** a fitting evaluation function (potential energy) of a model of the mitral annulus in a fitting model prepared in consideration of the physical shapes of the heart and the mitral annulus is optimized by the replica exchange method and extended simulated annealing method.

FIG.4

## EP 1 832 233 A1

**Description**

TECHNICAL FIELD

The present invention relates to a method and device for obtaining measurements or data of a cardiac valve to be used for clinical purposes. More specifically, the present invention relates to a method and device for automatically extracting a clear three-dimensional image of a cardiac valve, from which various measurements or data regarding the cardiac valve can be obtained.

BACKGROUND ART

Mitral regurgitation (mitral valve insufficiency) frequently occurs among valvular diseases, and in the case of severe regurgitation, left-sided cardiac failure occurs. Therapy for a severe mitral regurgitation is basically a surgical treatment, and conventionally, a mitral-valve replacement operation using an artificial valve has been performed. However, such an operation causes various problems after replacement with an artificial valve, such as deterioration of the cardiac function, and complication associated with an anticoagulation treatment. Therefore, in recent years, a mitral valve plasty, which maintains the original valve, has been widely performed.

The mitral valve plasty is a surgical method of selectively reconstructing a portion of the valve causing the regurgitation, among the mitral annulus, the mitral leaflet, the chordae tendineae, etc. In order to successfully perform such an operation, identification of etiology and accurate preoperative diagnosis of the lesion must be performed using echocardiography.

However, in the echocardiography widely used at the present, diagnosis is performed by use of a two-dimensional image, and therefore, it has been difficult to find the anatomical and positional relations between the mitral valve, which has a complex three-dimensional structure, and the surroundings thereof. That is, a two-dimensional image is insufficient, and three-dimensional image diagnosis is desired so as to grasp the three-dimensional structure of the functional complex of the mitral valve (mitral valve mechanism), which is constituted by the mitral annulus curved in the form of a saddle, the mitral cusp and leaflet having exquisite curves, and a supporting tissue located below the valve and extending from the chordae tendineae to the papillary muscle and the left ventricular.

Through use of a recently developed three-dimensional echocardiographic device, it becomes possible to scan the entire heart in real time conveniently in a noninvasive manner and capture an image thereof. A three-dimensional echocardiographic image allows observation of the structure of the cardiac muscle, valve, etc., as if a surgeon were actually observing the heart. Therefore, it is expected to realize preoperative diagnosis that is more detailed as compared with the conventional diagnosis performed on the basis of a two-dimensional image.

However, three-dimensional analysis and measurement through use of a three-dimensional image is still difficult, and the actual specific configuration and positional relation cannot be quantized. Therefore, presently, three-dimensional echocardiography has not been put in actual use for clinical purposes.

Non-Patent Document 1: Hiromitsu Yamada, "Recognition of Echocardiogram by Cooperation of Global Extraction and Local Tracking," [online], Bulletin of Electrotechnical Laboratory Vol. 62, No. 7 [Searched on December 22, 2005], Internet <http://www.etl.go.jp/jp/results/bulletin/pdf/62-7/yamada72.pdf>

DISCLOSURE OF THE INVENTION

PROBLEMS TO BE SOLVED BY THE INVENTION

Since CT and MRI apparatuses are large and expensive, they cannot be utilized by every hospital. In contrast, since an echocardiograph utilizing ultrasonic waves is small and can handy, it is widely used. Therefore, an echocardiographic (cardiac ultrasonic) inspection is indispensable for diagnosis and treatment of diseases of the circulatory system such as heart disease or hypertension. Information which in the past could be obtained only through cardiac catheterization can be grasped instantaneously without causing pain to the patient. Further, as a result of a reduction in the weight of the device, medical doctors can now carry the echocardiograph as they carry a stethoscope, whereby they can make a diagnosis at the site of visit.

An echocardiogram; e.g., an M-mode, two-dimensional, or a Doppler echocardiogram, is obtained by use of an echocardiograph. The M-mode echocardiogram provides graphic recording of motion of the cardiac structure with time, wherein motions of the valve, ventricular wall, aorta, etc. are depicted to present respective characteristic patterns. In the case of the two-dimensional echocardiogram, a two-dimensional tomogram (B mode) is obtained through high-speed scanning of an ultrasonic beam.

High-speed mechanical scanning and electronic scanning are used for scanning an ultrasonic beam. Through tomography, the cardiac configuration or cardiac motion can be observed conveniently, and thus, tomography is useful for determining whether or not any abnormality is present, and for diagnosing the site and extent of such abnormality. Examples of Doppler echocardiography include the pulse Doppler method, the continuous-wave Doppler method, Doppler tomography, the two-dimensional blood-flow imaging method, and the color Doppler method, which are applied not only to qualitative diagnosis through examination of an anomalous blood flow within the heart cavity, such as stenotic flow and valve regurgitation, but also to quantitative diagnosis such as blood flow measurement and pressure estimation, and evaluation of the cardiac function.

However, not all problems can be solved by use of echocardiogram. A conventional cardiac-valve automatic extraction device which employs the window method in which judgment is performed on the basis of a threshold value or the edge extraction method for extracting a location where intensity changes greatly causes erroneous recognition frequently. An echo image show unclear boundaries, unlike an image obtained by use of CT, MRI, or the like which shows clear boundaries. The window method or the edge extraction method can be applied to images such as those obtained by use of CT, MRI, or the like which have clear boundaries, but cannot be applied to images which do not have clear boundaries such as echocardiogram.

In addition to the above-described window method and edge extraction method, there has been proposed a method of obtaining a contour image from an image showing unclear boundaries, by modeling a cardiac valve by means of fitting of a curve, and applying a proper optimizing method. However, the Newton method and the steepest descent method cannot be applied to a complex figure. Further, even when a GA (genetic algorithm), SA (simulated annealing), or a like method is used with an increased degree of freedom, there arises a problem in that a local minimum value functions as a "trap" for a solution, and therefore, finding an optimal solution is difficult.

In view of the foregoing, an object of the present invention is to provide a method and apparatus for acquiring information regarding the cardiac valve required in the clinical field, such as the tenting volume, tenting area, tenting height of the mitral valve of the heart, the area, the circumferential length, and height (the difference between the highest portion and the lowest portion) of the mitral annulus, etc.

Data of the cardiac valve is automatically extracted from an echocardiogram acquired by use of an echocardiograph, to thereby produce a clear three-dimensional image of the valve, and required quantity-related items are measured from this image. A method and device used in the present invention can realize not only automatic extraction of a clear three-dimensional image of the cardiac mitral annulus in the echocardiogram, but also reproduction of a tissue boundary not appearing on the echo image. That is, the present invention provides a method and device for automating the function of identifying the cardiac valve, which conventionally has been recognized only by the eyes of a skilled doctor.

MEANS FOR SOLVING THE PROBLEMS

In order to solve the above-described problems, the invention described in claim 1 provides a method of automatically extracting a three-dimensional cardiac-valve image for measuring clinically required data regarding the cardiac valve, in which method a three-dimensional echocardiogram is created from two-dimensional echocardiograms obtained through scanning by means of an echocardiograph, and the three-dimensional cardiac-valve image is automatically extracted from the three-dimensional echocardiogram by computer processing, the method being characterized in that a fitting evaluation function (potential energy) of a model of the mitral annulus in a fitting model prepared in consideration of the physical shapes of the heart and the mitral annulus is optimized by the replica exchange method and extended simulated annealing method.

The invention described in claim 2 provides a device for automatically extracting a three-dimensional cardiac-valve image for measuring clinically required data regarding the cardiac valve, in which method a three-dimensional echocardiogram is created from two-dimensional echocardiograms obtained through scanning by means of an echocardiograph, and the three-dimensional cardiac-valve image is automatically extracted from the three-dimensional echocardiogram by computer processing, the device being characterized by comprising means for optimizing, by the replica exchange method and extended simulated annealing method, a fitting evaluation function (potential energy) of a model of the mitral annulus in a fitting model prepared in consideration of the physical shapes of the heart and the mitral annulus.

Specifically, in the present invention, the following procedure is performed for extraction of the data representing the mitral annulus (hereinafter, may be simply referred to as "extraction of the mitral annulus") and fitting thereof. The mitral annulus extraction processing includes the following two steps. First, a fitting model created in consideration of the physical shape of the heart is prepared, and a portion of the cardiac muscle having a high intensity is fitted thereto. Subsequently, on the fitted shape, a portion which is likely to be the mitral annulus is searched.

A cylindrical network structure formed of an elastic material can be used as a model of the mitral annulus. For example, a total of 1,600 control points (40 (circumferential direction) x 40 (height direction)) are provided, and these control points are connected with one another by springs having proper spring forces. At this time, to the extent possible, the control points are set at locations of high intensity. The fitting evaluation function (potential energy) of this cylindrical mitral annulus model is optimized by the replica exchange method and extended simulated annealing method.

The replica exchange (RE) method is widely used for elucidating the three-dimensional molecular structure of a protein or the like. In this method, the global system consisting of a plurality of equivalent systems (replicas) having no interaction is considered, different temperatures (energies) are allotted to the replicas (copies), and the same molecules are initially disposed in all the replicas. Metropolis simulation is individually performed in each replica system, and the molecular arrangement is periodically exchanged between adjacent replicas.

Moreover, by means of simulated annealing (SA), annealing from high temperature (high energy) to low temperature (low energy) is performed to find the optimal solution. In this method, a point (optimal solution) at which the potential surface of the energy of a structure becomes minimum (or local minimum) is found, and a final molecular structure is determined as a stable structure.

For exchange of the molecular arrangement between the replicas, the Monte-Carlo method in which exchange is performed randomly, a genetic algorithm (GA) in which exchange is performed between close molecules (between adjacent molecules) as in the case of gene recombination, or a like method is used. When the molecular structure is modeled, molecules are considered as points, and Coulomb force, spring interaction, etc. act between adjacent molecules, and the sum of these forces is represented as an intramolecular potential energy.

Exploration of the mitral annulus is performed in accordance with the following rules.

- Explore locations of high intensity to a possible extent
- Explore locations where the second derivative is positive as viewed from the lower side to the upper side (recessed portion)
- Explore locations which are not excessively separated from adjacent control points of the mitral annulus.

Proper evaluation functions are defined for these rules, and optimization is performed in a similar manner. A structure which shows the minimum potential energy is finally extracted as representing a portion which is possibly the mitral annulus. For the case where automatic extraction of the mitral annulus has failed, a route for manual correction may be provided.

EFFECTS OF THE INVENTION

The device of the present invention has the following advantageous features. (1) The device can provide three-dimensional display and quantitative analysis of the mitral valve complex, which have been impossible when conventional two-dimensional echocardiograms are employed. (2) Whereas the conventional reconstruction of a three-dimensional image from two-dimensional images is a laborious and time-consuming process, the device of the present invention requires only a short time (currently, about 15 minutes) before completion of the entire process, including three-dimensional analysis of the mitral valve (collection of echo images, tracing of the images, reconstruction of a three-dimensional image, and quantitative analysis of three-dimensional data).

Three-dimensional quantitative analysis using a three-dimensional echocardiogram has never been realized, and the present inventors are the first in the world to have accomplished the present invention. In particular, at present, attention is drawn worldwide to the elucidation of the mechanism of "functional mitral regurgitation" which is caused by the malfunction of the papillary muscle or the left ventricle even though the cusp and leaflet of the mitral valve have no anomaly, as well as to the development of therapy therefor. Studies on these themes, which have relied on analysis of two-dimensional echocardiogram images, are expected to greatly progress because three-dimensional analysis has become possible. The device of the present invention can be used for preoperative diagnosis and surgical treatment of mitral regurgitation, and is therefore clinically very useful.

BRIEF DESCRIPTION OF THE DRAWINGS

FIG. 1 is a set of views for explaining a method of capturing 18 images of the heart in a contraction phase by means of an echocardiograph and reconstructing the leaflet and annuls of the mitral valve in the form of a 3D image.

FIG. 2 is an explanatory illustration showing creation of a 3D image from 2D images.

FIG. 3 is a set of views showing a three-dimensional cardiac valve image of the leaflet and annulus of the mitral valve of a healthy person, which image is reconstructed by use of a device of the present invention, wherein (A) is a pair of perspective views showing the appearance of the leaflet and annulus of the mitral valve, (B) shows a top view of the leaflet of the mitral valve as viewed from the LV, and a side view of the leaflet, (c) shows top and side views of the leaflet of the mitral valve obtained by correcting the previous views.

FIG. 4 is a set of views showing a three-dimensional cardiac valve image of the leaflet and annulus of the mitral valve of a person suffering from ischemic MR, which image is reconstructed by use of the device of the present invention, wherein (A) is a pair of perspective views showing the appearance of the leaflet and annulus of the mitral valve, (B) shows a top view of the leaflet of the mitral valve as viewed from the LV, and a side view of the leaflet, (c) shows top and side views of the leaflet of the mitral valve obtained by correcting the previous views.

FIG. 5 is a diagram showing the distribution of the maximum tenting sites of 12 patients suffering from local anemia MR.

FIG. 6 is an explanatory diagram showing control points and elastic springs.

FIG. 7 is an explanatory diagram showing evaluation functions and integration areas.

FIG. 8 is an explanatory diagram showing a potential surface and local minimums.

BEST MODE FOR CARRYING OUT THE INVENTION

A best mode for carrying out the present invention will be described with reference to the drawings. In the description hereinbelow, the following abbreviations and acronyms will be used.

MR = mitral regurgitation
3D = three-dimensional
2D = two-dimensional
LV = left ventricle
LA = left atrium
ROA = regurgitation orifice area
EF = ejection fraction
PISA = proximal isovelocity surface area
EDV = end-diastolic volume (capacity)
ESV = end-systolic volume (capacity)

Two-dimensional echocardiography provides the following examinations. When standard 2D echocardiography is performed for all subjects, the end-diastolic volume (EDV) and end-systolic volume (ESV) of each subject can be measured by a modified Simpson's method (wherein the entire left ventricle is approximated as a stack of cylinders). As a result, the ejection fraction (%) can be calculated by an equation 100x(EDV-ESV)/EDV. MR is evaluated by color Doppler echocardiography. The degree of MR can be quantized by the PISA method using ROA. However, in order to perform mitral regurgitation diagnosis or mitral valve operation, the accurate position of the mitral annulus must be determined, and a three-dimensional valve image is required. The device of the present invention precisely identifies the mitral valve through execution of the following steps, and reproduces a clear image thereof.

In order to obtain a three-dimensional image (volumetric image), an image (full volume mode) of a subject (in a cardiac apex view) for capacity measurement through the thoracic cavity is obtained by use of a real-time 3D echocardiogram system. The frame rate for capacity measurement is 16 to 22 frames per sec at a depth of 12 to 16 centimeters (the frame rate depends on the depth). Before acquisition of a complete three-dimensional image, adjustment is performed such that a probe is located at the top of a center portion of the mitral valve in the 2D images. All the three-dimensional images are recorded on a compact disc in a digital format, and are transferred to a personal computer for offline analysis.

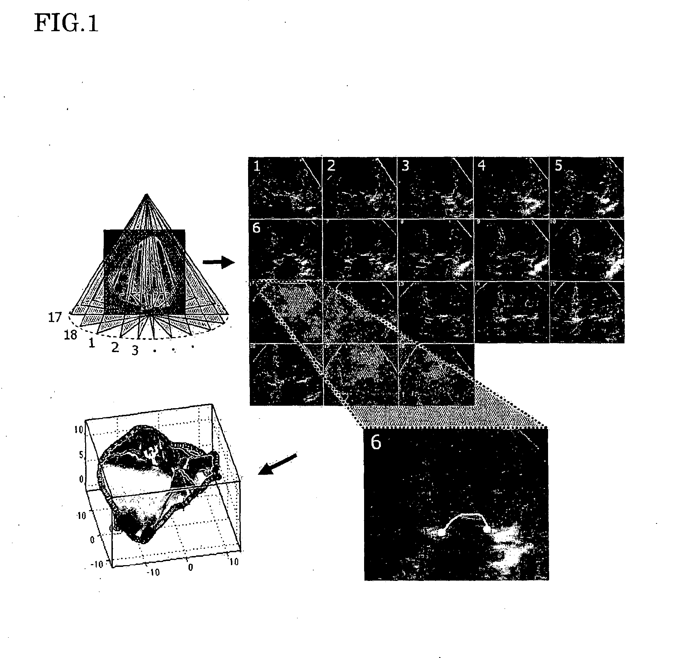

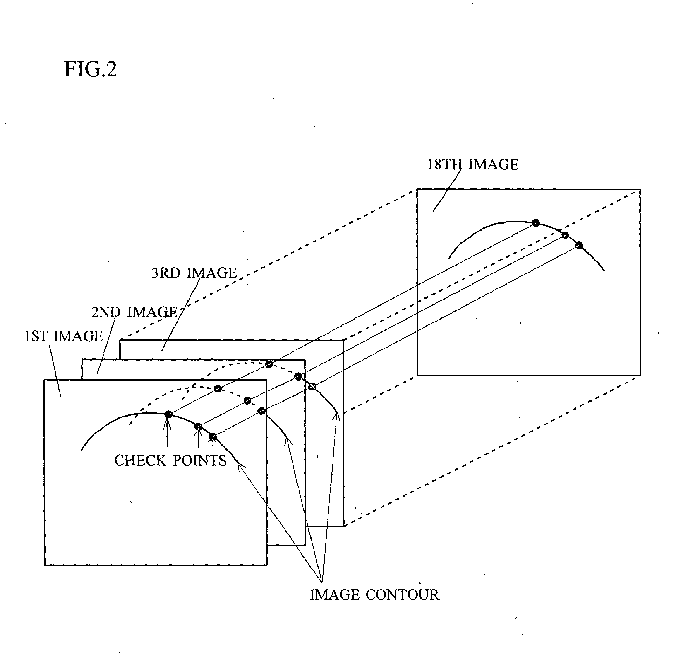

FIG. 1 shows a process of automatically capturing (scanning) 18 radial plane images at equal intervals by use of a three-dimensional echocardiograph, and forming a three-dimensional (3D) image on the basis of the plane images. The annulus and leaflet of the mitral valve are manually marked for each plane image obtained through scanning during the contraction phase of the heart. A 3D image of the annulus and leaflet of the mitral valve is reconstructed from these data. A specific process is shown in FIG. 2.

As shown in FIG. 2, plane images obtained through scanning of an object are sequentially arranged, corresponding check points (respective points on the images which correspond to one another) are connected by lines, and smoothing and rendering are performed, thereby producing a three-dimensional image of the object (FIG. 2 shows a case where 18 frames are used). However, as described in the "BACKGROUND ART" section, a clear contour cannot be obtained from an echo image, unlike the case of MRI or CT. In particular, since the mitral annulus has a complex and intricate anatomy, the device of the present device employs a fitting model for extracting images of the mitral annulus. For such extraction, a portion of the cardiac muscle having a high intensity is fitted in consideration of the physical shape of the heart. Further, the location where the mitral annulus is likely to be identified is searched on the fitted shape. A fitting model, an example of which will be described below, is used for identifying the mitral annulus.

In this example, a cylindrical network structure formed of an elastic member is used as a fitting model. A total of 1,600 control points (40 (circumferential direction) x 40 (height direction)) are provided, and these control points are connected with one another by proper springs. The control points are set at locations of high intensity to a possible extent. A plurality of such structures (replicas) are prepared such that these replicas have different intensities. Intensity is used as potential energy, and a structure whose potential energy becomes most stable (assumes the minimum value) is determined. The method used for this purpose is extended simulated annealing, called the replica exchange method. That is, the process is started from a location were the intensity is high, the control points are exchanged between the replicas, and the potential energy is obtained each time. Through such simulation, a structure (stable structure) which has the minimum potential energy is extracted as the shape of the structure (mitral annulus) (optimization).

Notably, the main rules used for automatic extraction and exploration of the mitral annulus are as follows.

- Explore locations of high intensity to a possible extent
- Explore locations where the second derivative is positive as viewed from the lower side to the upper side (recessed portion)
- Explore locations which are not excessively separated from adjacent control points of the mitral annulus.
  When proper evaluation functions are defined for these rules and optimization is performed as in the case of exploration of the shape of the heart, a portion which is possibly the mitral annulus is identified. For the case where automatic extraction of the mitral annulus has failed or the result of the extraction is ambiguous, a route for manual correction is provided.

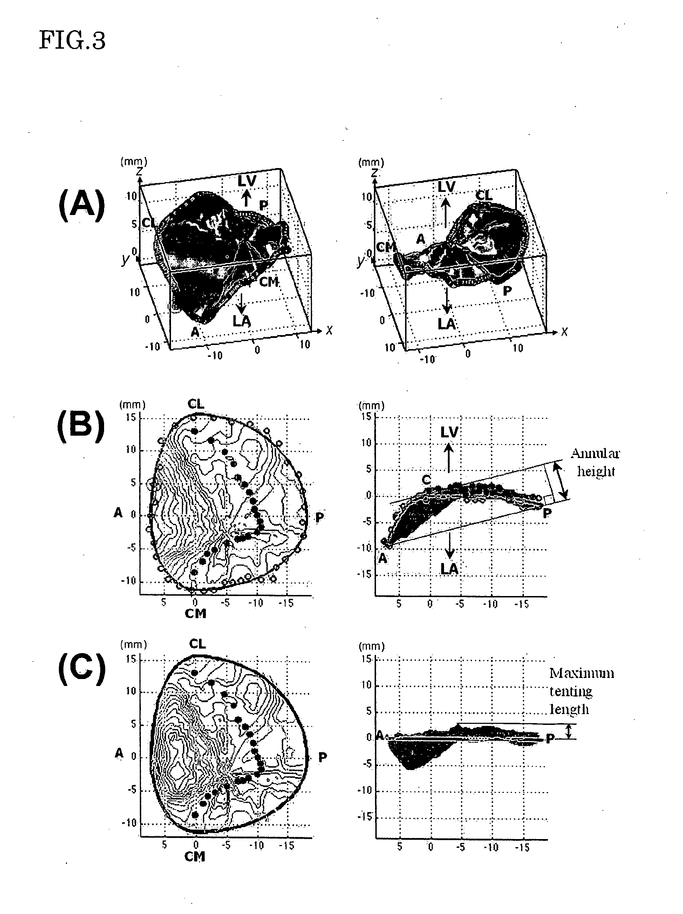

FIG. 3 shows the mitral annulus extracted in the above-described manner. In the following description, the extracted mitral valve will be called "leaflet," and the root of the leaflet will be called "mitral annulus." Further, inflation of the leaflet in the manner of setting up a tent or a swell of the leaflet will be called "tenting." Blood having been cleaned in the lungs flows into the left atrium (LA), and is fed from the LA to the left ventricle (LV) via the mitral valve. The blood is then fed from the LV to the entire body via the aorta. Therefore, the pressure in the LV becomes higher than that in the LA.

When a physical or functional disorder occurs at the valve, stenosis or ischemia occurs. For example, if the mitral valve does not open completely and sufficient blood is not fed to the LV, stenosis occurs. In contrast, if the mitral valve slackens and mitral valve insufficiency occurs, the reverse flow of blood from the LV to the LA occurs. In this case, a sufficient amount of arterial blood is not supplied to the body, and ischemia occurs. In recent years, in an increasing number of cases, mitral valve insufficiency caused by slackening of the mitral valve is treated by means of valvuloplasty, without use of an artificial valve. For such valvuloplasty, obtaining the accurate shape of the mitral valve is important, and thus, acquiring a three-dimensional image of the cardiac valve by use of the device of the present invention is effective.

The symbols shown in the drawings have the following meanings.

| | |
|---|---|
| A: | anterior |
| P: | posterior |
| CL: | antero-lateral commissure |
| CM: | postero-medial commissure |
| LV: | left ventricle |
| LA: | left atrium |
| annular height: | height of the mitral annulus (the degree of curvature) |
| tenting length: | length of tenting |

FIG. 3 shows a three-dimensional image of the leaflet of the mitral valve of a healthy person obtained through three-dimensional-cardiac-valve-image extraction performed by the device of the present invention, as well as the shape of the leaflet. Section (A) of FIG. 3 show 3D images of the leaflet as viewed from different directions. In these images, the annulus (root portion) of the mitral valve assumes a "saddle-like shape." Although the leaflet of the mitral valve slightly curves into the LV, it appears to be generally flat.

Section (B) of FIG. 3 shows actual 3D tenting images. The mitral annulus is illustrated in its outline for 3D measurement. The left-hand image shows the shape of the leaflet as viewed from the LV, and the degree of tenting is represented by contour lines. The right-hand image shows the shape of the leaflet as viewed from a horizontal direction, and enables accurate measurement of the degree of tenting of the mitral annulus and leaflet. The circumference and area of the annulus of the mitral valve can be measured from these 3D data. The height of the mitral annulus in the right-hand image represents the degree of curvature of the mitral annulus. Black dots in the images show an engagement line (the commissure portion of the valve; that is, the location where the anterior and the posterior engage when the LV contracts). In the case of a healthy person, when the LV contracts, the anterior and the posterior properly engage while being supported by the chordae tendineae of the mitral valve, whereby the blood flow from the left ventricle to the left atrium is stopped. This engagement line is represented by the black dots.

Section (C) of FIG. 3 show corrected 3D tenting images. Curved thick lines in the images represent the annulus of the mitral valve, which is smoothly drawn on a plane with the distance from the annular surface to the leaflet maintained constant. The left-hand image shows the shape of the leaflet as viewed from the LV, and the degree of tenting is represented by contour lines. The right-hand image shows the shape of the leaflet as viewed from a horizontal direction, allowing quantitative measurement of the degree of tenting of the annulus of the mitral valve. The maximum tenting length, the average tenting length, and the tenting volume can also be measured from these 3D data. Notably, black dots represent the engagement line.

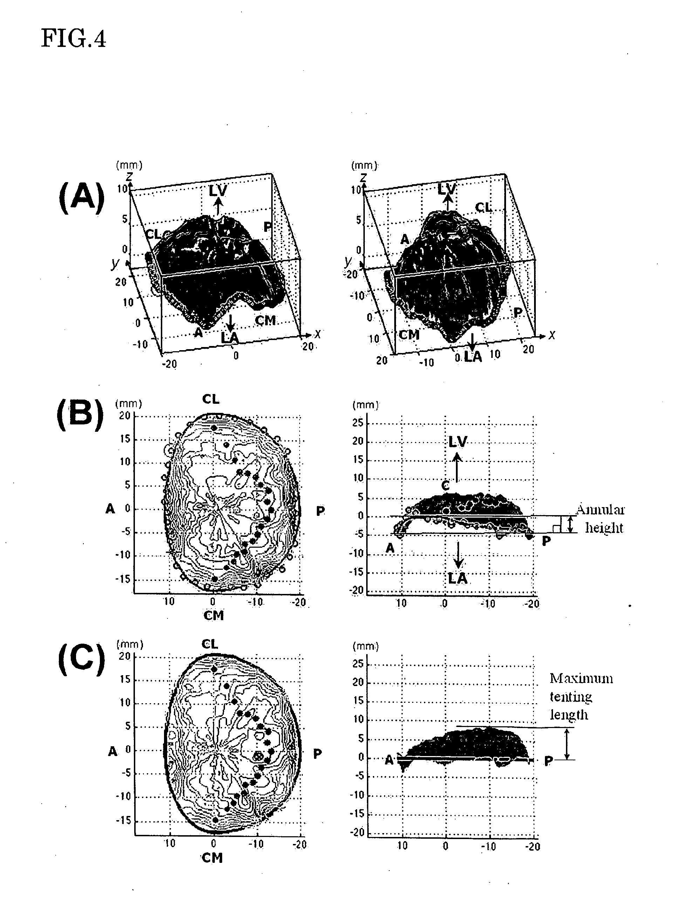

FIG. 4 is a three-dimensional image of the annulus of the mitral valve, showing the leaflet of the mitral valve of a patient suffering from ischemic mitral regurgitation (MR). Section (A) of FIG. 4 show 3D images of the leaflet as viewed from different directions. As is clear from the appearance, the annulus of the mitral valve has a smoothed or flattened shape, due to tenting. Further, the curved leaflet assumes a convex shape, and generally invades into the LV.

Section (B) of FIG. 4 shows actual 3D tenting images. These images show that the entire leaflet of the mitral valve apparently inflates toward the LV, and the height of the mitral annulus is smaller than that in the case of the healthy person. Further, the annulus of the mitral valve is expanded. Notably, black dots represent the engagement line.

Section (C) of FIG. 4 shows corrected 3D tenting images. As is apparent from the left-hand image, the leaflet of the mitral valve is substantially symmetrical with respect to A-P when viewed in the annulus of the mitral valve. As can be understood from the right-hand image, the maximum tenting length is greater than that in the case of a healthy person. Black dots represent the engagement line. When these images are color-displayed, a green mark (a portion lightly printed in the right-hand image) shows the maximum tenting site of the leaflet. In the case of this patient, the maximum tenting site is located at the center of the leaflet anterior A (a location having the maximum height indicated by the contour lines in the left-hand image <a position corresponding to the peak of a mountain>).

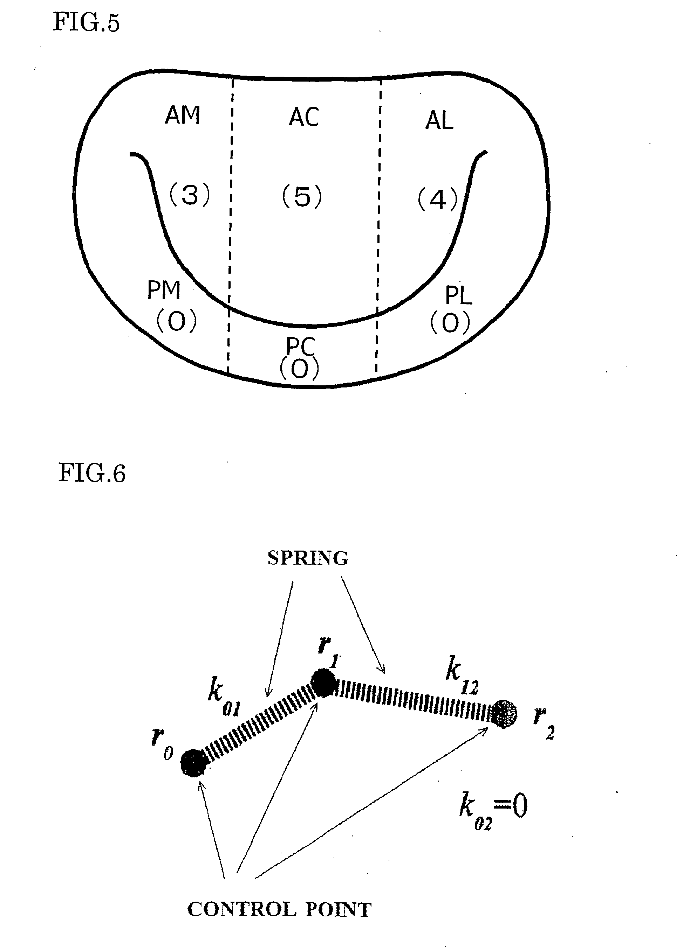

FIG. 5 is a diagram showing the results of an investigation of the maximum tenting sites of 12 patients suffering from local anemia MR, wherein the results are represented on the leaflet by the distribution of the patients. In FIG. 5, English letter "A" represents the anterior, "P" represents the posterior, "L" represents a lateral portion, "C" represents a central portion, and "M" represents a medial portion. Further, each parenthesized number represents the number of patients. As shown in FIG. 5, for all the 12 patients, the maximum tenting site was located in the leaflet front portion. Specifically, the maximum tenting sites of three patients were located in AM, the maximum tenting sites of five patients were located in AC, and the maximum tenting sites of four patients were located in AL.

The 12 patients suffering from ischemic MR include three patients each suffering from a single vascular disease, six patients each suffering from two vascular diseases and three patients each suffering from three vascular diseases. Severe LV functional disorder was found in a wide range (EF: $33.9 \pm 9.1\%$; width: 18% to 47%). ROA was $0.29 \pm 0.15$ $cm^2$ (ranging from 0.15 to 0.62 $cm^2$). Through comparison with 10 experiment controls, any difference that distinguishes the patients suffering ischemic MR (in terms of age, sex, or body surface area) was not found. However, in the case of the patients suffering ischemic MR, the LV has a considerably increased volume as compared with the case of healthy persons.

As described above, a software system for producing an image for real-time 3D echocardiography, which has been developed by making use of the three-dimensional-cardiac-valve-image acquiring method of the present invention, was able to perform quantitative measurement of 1) a 3D geometric anomaly of the leaflet and annulus of the mitral valve; 2) the maximum tenting site of the leaflet of the mitral valve; and 3) the mitral valve tenting and the geometric anomaly of the mitral annulus of a patient suffering from ischemic MR.

The fitting model used in the present invention will be described with reference to a specific example. Because of the characteristics of an echo measurement apparatus, noise and shadows appear on an obtained image. Therefore, it is difficult to obtain an accurate image of an organ of interest only from the information of the obtained image. A medical doctor knows the ideal image of the actual organ, and, in his head, combines the ideal image with echo images at different angles and times and then draws a boundary line of the organ by complementing the unclear echo images. By use of physical modeling, the image-complementing work that has been performed in the physician's head can be performed on a computer.

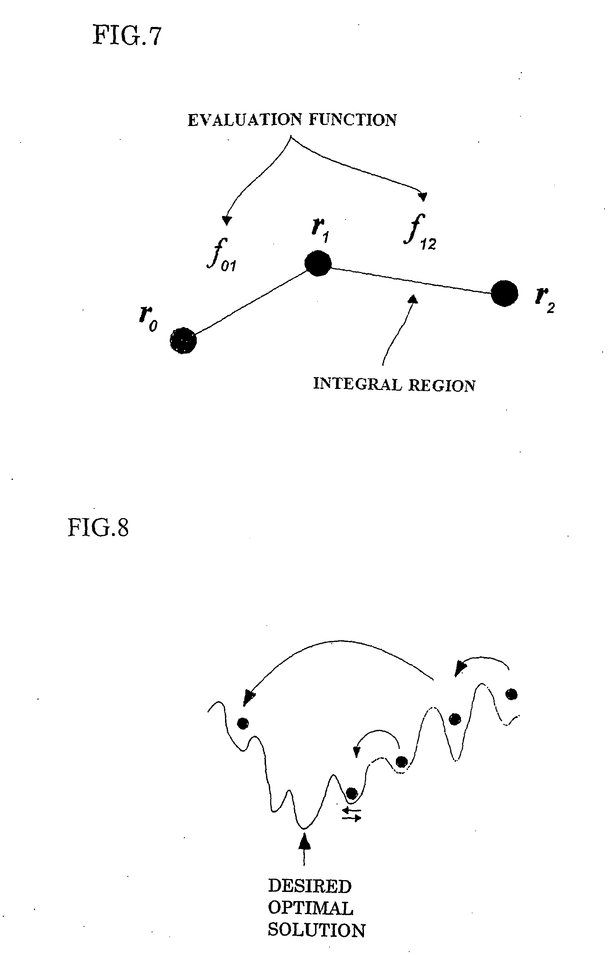

Construction of a model on a computer is performed by use of springs connecting control points and boundary evaluation functions between the control points as shown in FIGS. 6 and 7. The springs between the control points maintain the physical structure of the organ. Meanwhile, the boundary evaluation functions acquire boundary information of the organ from an image thereof.

A potential function, which is the sum of the elastic energy of each spring and the evaluation energy produced by the boundary evaluation, is used for evaluation of the model. The position of an i-th control point is represented by $r_1$, and a set of control points $r_1$, $r_2$, ... $r_N$ is represented by $r^N$. At this time, the elastic energy function $S(r^N)$ of the springs is defined as follows.

[Eq. 1]

$$S(r^N) = \sum_{i<j}^{N} \left[ \left( \frac{\sigma}{|r_j - r_i|} \right)^6 + k_{ij} |r_j - r_i|^2 \right]$$

Here, $K_{ij}$ represents the elastic strength of the spring, and is empirically determined from the strength of the tissue between the control points and the like. $K_{ij}$ is set to zero when the relevant control points are not connected. $\sigma$ is a control point elimination radius which is selected to prevent mutual overlapping of the control points. At this time, the natural length of the spring is represented as follows.

[Eq. 2]

$$3^{1/8}\sigma^{3/4}\kappa_{ij}^{1/8}$$

These parameters are set such that the energy becomes the lowest when the physical shape is ideal.

[Eq. 3]

$$E(r^N)$$

An evaluation energy function of Eq. 3 is defined as shown in Eq. 5 by use of a function of Eq. 4, which sends back the intensity at the vector r point of the image.

[Eq. 4]

$$M(r)$$

[Eq. 5]

$$E(r^N) = \sum_{i<j}^{N} \int_{r_i \to r_j} c_{ij} f_{ij}(M, p, \vec{r}_{ij}) dp$$

Here,

[Eq. 6]

$$\vec{r}_{ij}$$

is a vector having a length of 1 defined by the following equation.

[Eq. 7]

$$\vec{r}_{ij} = (r_j - r_i)/|r_j - r_i|$$

$c_{ij}$ represents a coupling constant.
**[Eq. 8]**

$$f_{ij}(M, p, \vec{r})$$

is an evaluation function (which will be described later) between control points i and j, and the line integration is defined by the shortest route between $r_i$ and $r_j$.
A function which reflects the physical property is empirically chosen as the evaluation function between the control points. For example, a function which recognizes, as a boundary, a location where the intensity changes greatly (the energy drops in the vicinity of the boundary) can be written as follows.
**[Eq. 9]**

$$f_{border}(M, p, \vec{r}) = -|\nabla M(p) \cdot \vec{r_\perp}| = -\sqrt{|\nabla M(p)|^2 - (\nabla M(p) \cdot \vec{r})^2}$$

Here,
**[0065]**

**[Eq. 10]**

$$\vec{r_\perp}$$

is a vector having a length of 1 perpendicular to the following vector.
**[Eq. 11]**

$$\vec{r}$$

**[Eq. 12]**

$$\nabla M(p)$$

represents the slope of the function M.
Similarly, the following functions can be used as the evaluation function.
[Eq. 13]

$$\text{Cavity:} \quad f_{void}(M, p, \vec{r}) = -M(p)$$

$$\text{Tissue:} \quad f_{tissue}(M, p, \vec{r}) = M(p)$$

$$\text{No evaluation function:} \quad f_{none}(M, p, \vec{r}) = 0$$

An energy function as shown below is finally defined by combining these functions.
[Eq. 14]

$$F(r^N) = W_s \cdot S(r^N) + W_E \cdot E(r^N)$$

$W_S$ and $W_E$ are weights for the elastic energy and the evaluation energy, and are adjusted depending on whether importance is attached to the structure or the boundary evaluation. A set of control points represented as follows and minimizing the value of the function F is searched.
[Eq. 15]

$$r^N$$

Thus, the boundary of the tissue can be extracted. The characteristic of this function F resides in that the boundary can be searched by the function E, while the physical shape represented by the function S is maintained. Even when some noise and shadows are present on an echo image, by virtue of the complementation by the physical shape, a plausible boundary of the tissue can be extracted even if the shape cannot be guessed from the image only.
Optimization processing according to extended simulated annealing will be described. In order to accurately determine the boundary of the tissue, a large number of control points are required. However, since the evaluation function is non-linear, when the number of the control points increases, finding the minimum point of the evaluation function becomes difficult. Even when the Newton method, the steepest descent method, GA (genetic algorithm), or SA (simulated annealing), which are typical optimization methods, is used, the processing is easily trapped at the local minimum, and thus, the optimum solution cannot be reached.
Extended simulated annealing is a strong optimization method which has drawn attention in recent years in the fields of physics and chemistry and which is used for solving the spin glass phenomenon and the problem of protein folding. Extended simulated annealing is a calculation method which can efficiently solve a complex optimization problem having multiple degrees of freedom. We optimized the evaluation function by making use of the replica-exchange Monte-Carlo method, which is one type of extended simulated annealing.
First, simulated annealing by the Monte-Carlo method, which is the basis of the replica-exchange Monte-Carlo method, will be described. In the Monte-Carlo method, computer simulation is performed by making use of a probabilistic algorithm.
**[Eq. 16]**

$$r_1, r_2, \ldots r_N$$

The above set of control points in the first step is represented as follows.
**[Eq. 17]**

$$r_0^N$$

Next, one control point is randomly selected from these control points, and shifted in a random direction/amount as shown below.
**[Eq. 18]**

$$\Delta r$$

That control point set is represented as follows.
**[Eq. 19]**

$$r_0^{t^N}$$

The evaluation energy in the initial step and that after the shift can be written as follows.
**[Eq. 20]**

$$E(r_0^N), \quad E(r_0^{t^N})$$

The set of shifted control points is employed in the next step at the following probability.
**[Eq. 21]**

$$E(r_0^N) > E(r_0^{t^N})$$

When the above relation is satisfied, the set of shifted control points is employed.
[Eq. 22]

$$E(r_0^N) < E(r^{t_0^N})$$

When the above relation is satisfied, the set of shifted control points is employed at a probability represented as follows.
[Eq. 23]

$$\exp[-\beta E(r^{t_0^N}) + \beta E(r_0^N)]$$

When the set of shifted control points is employed, the set is stored as $r_1^N$ as follows, and processing proceeds to the next step.
[Eq. 24]

$$r_1^N = r^{t_0^N}$$

When the set is not employed, $r_0^N$ is stored as $r_1^N$ as follows.
[Eq. 25]

$$r_1^N = r_0^N$$

Subsequently, processing proceeds to the next step.
Here, $\beta$ is a parameter for determining the degree of optimization of the system, and the parameter is known in statistical thermodynamics to be obtained by $\beta = 1/k_B T$, where $k_B$ is the Boltzmann constant, and T is temperature. When the value of $\beta$ is sufficiently large (temperature is low), the value of the evaluation function decreases with the progress of the simulation. Meanwhile, when the value of $\beta$ is small (temperature is high), the value of the evaluation function can increase, so that the function exhibits a large variation.
The evaluation function can be expressed as a plane (potential plane) in a 3N+1 dimensional space, and the simulation proceeds while jumping from one to another of the local minimums on the surface.
In order to perform an optimization search by the Monte-Carlo method, the value of $\beta$ is first decreased so as to bring the set of control points into a random state and mix them, and is then increased gradually so as to converge the value of the evaluation function. After the value of $\beta$ is increased sufficiently, the simulation procedure is performed for a while so as to search a set of control points:
[Eq. 26]

$$r_0^N$$

which minimizes the value of the evaluation function. When the number of control points is small or when the evaluation function is not complex, the optimum point can be found by this method. However, since the potentially surface generally has a complex shape, when the temperature is simply decreased, the simulation is shortly trapped at a local minimum, and optimization cannot be performed to a sufficient degree even when the simulation is performed for a long period of time (see FIG. 8).

In the replica exchange Monte-Carlo method, the above-described Monte-Carlo simulation is simultaneously performed at different temperatures so as to prevent the simulation from being trapped at a local minimum, to thereby efficiently perform the optimization. M sets of control points (replicas) are prepared. Simulation is performed for the m-th set of control points:

$$\mathbf{[Eq.\ 27]}$$

$$r_m^N$$

while using a parameter $\beta_m$. Here, the temperature parameter $\beta_m$ for each replica are arranged in descending order of temperature; e.g., $\beta_m < \beta_{m+1}$. At proper step intervals, the positions of control points are exchanged between the replicas by the following method.

$$\mathbf{[Eq.\ 28]}$$

$$\Delta = (\beta_{m+1} - \beta_m)(E(r_m^N) - E(r_{m+1}^N))$$

When $\Delta < 0$, the positions are exchanged.
When $\Delta > 0$, the positions are exchanged at a probability of $\exp(-\Delta)$.

By virtue of this temperature exchange method, even when a set of control points whose temperature is low is trapped at a local minimum, such a set is exchanged with a set whose temperature is adequately high so as to escape from the local minimum. When the parameter is properly set, the optimization proceeds with time.

In general, when the number of replicas is increased so as to decrease the difference between adjacent $\beta_m$ values, the optimal solution can be found more easily. However, an increase in the number of replicas results in an increase in the calculation cost. Therefore, the number of replicas must be adjusted so as to maximize the calculation efficiency while investing the variance of the evaluation function. In order to increase the calculation efficiency, there must be created a state in which exchange occurs between the replicas at a sufficiently large frequency, and one replica can randomly walk in the temperature space. For such a purpose, the number of replicas and the value of $\beta_m$ must be adjusted such that the variances of the evaluation functions of adjacent replicas overlap at the same area.

Although this method may require a large amount of labor for adjusting parameters and implementing the calculation algorithm, if the simulation is performed for a prolonged period of time, the optimal point can be found at a considerably high probability. Thus, unlike the cases where other optimization algorithms are employed, according to the present invention, once the parameters are adjusted, it is no longer necessary to perform a trial again and again while carefully selecting initial values. Therefore, an accurate boundary can be automatically extracted with almost no intervention by a human.

## Claims

1. A method of automatically extracting a three-dimensional cardiac-valve image for measuring clinically required data regarding the cardiac valve, in which method a three-dimensional echocardiogram is created from two-dimensional echocardiograms obtained through scanning by means of an echocardiograph, and the three-dimensional cardiac-valve image is automatically extracted from the three-dimensional echocardiogram by computer processing, the method being **characterized in that** a fitting evaluation function (potential energy) of a model of the mitral annulus in a fitting model prepared in consideration of the physical shapes of the heart and the mitral annulus is optimized by the replica exchange method and extended simulated annealing method.

2. A device for automatically extracting a three-dimensional cardiac-valve image for measuring clinically required data regarding the cardiac valve, in which method a three-dimensional echocardiogram is created from two-dimensional echocardiograms obtained through scanning by means of an echocardiograph, and the three-dimensional cardiac-valve image is automatically extracted from the three-dimensional echocardiogram by computer processing, the device being **characterized by** comprising means for optimizing, by the replica exchange method and extended simulated annealing method, a fitting evaluation function (potential energy) of a model of the mitral annulus in a fitting model prepared in consideration of the physical shapes of the heart and the mitral annulus.

FIG.1

FIG.2

18TH IMAGE

3RD IMAGE

2ND IMAGE

1ST IMAGE

CHECK POINTS

IMAGE CONTOUR

## FIG.3

# FIG.4

FIG.5

FIG.6

FIG.7

EVALUATION FUNCTION

$f_{01}$   $r_1$   $f_{12}$

$r_0$   $r_2$

INTEGRAL REGION

FIG.8

DESIRED
OPTIMAL
SOLUTION

<div align="center">

**INTERNATIONAL SEARCH REPORT**

</div>

| | International application No. |
|---|---|
| | PCT/JP2005/023797 |

A. CLASSIFICATION OF SUBJECT MATTER
*A61B8/00*(2006.01)

According to International Patent Classification (IPC) or to both national classification and IPC

B. FIELDS SEARCHED

Minimum documentation searched (classification system followed by classification symbols)
A61B8/00-8/15

Documentation searched other than minimum documentation to the extent that such documents are included in the fields searched
Jitsuyo Shinan Koho        1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)
Ichushi WEB

C. DOCUMENTS CONSIDERED TO BE RELEVANT

| Category* | Citation of document, with indication, where appropriate, of the relevant passages | Relevant to claim No. |
|---|---|---|
| P,A | Nozomi WATANABE et al., "Kyoketsusei Soboben Gyakuryu ni Okeru Soboben tenting no Keijo wa Shinkinkosoku Bui ni yori Kotonaru: Real Time Sanjigen Shin Echo-zu o Mochiita Teiryo Kaiseki", Journal of Cardiology, 10 August, 2005 (10.08.05), Vol.46, special extra issue 1, page 309 | 1,2 |
| A | Yasuo OGASAWARA et al., "Real Time 3D Choonpa Dansozo ni yoru Soboben Warittai Kozo no Hyoka", IEICE Technical Report, 07 July, 2004 (07.07. 04), Vol.104, No.179, pages 5 to 8 | 1,2 |

☐ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 March, 2006 (06.03.06) | 14 March, 2006 (14.03.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HIROMITSU YAMADA.** Recognition of Echocardiogram by Cooperation of Global Extraction and Local Tracking. *Bulletin of Electrotechnical Laboratory,* 22 December 2005, vol. 62 (7, <http://www.etl.go.jp/jp/results/bulletin/pdf/62-7/yamada72.pdf>